(19) Europäisches Patentamt
European Patent Office
Office européen des brevets

(11) EP 0 404 720 B1

(12) **EUROPÄISCHE PATENTSCHRIFT**

(45) Veröffentlichungstag und Bekanntmachung des Hinweises auf die Patenterteilung:
**28.02.1996 Patentblatt 1996/09**

(51) Int. Cl.[6]: **C07C 271/16**, A01N 47/12

(21) Anmeldenummer: **90810426.8**

(22) Anmeldetag: **12.06.1990**

(54) **Pestizides Mittel**

Pesticidal agent

Agent pesticide

(84) Benannte Vertragsstaaten:
**AT BE CH DE DK ES FR GB GR IT LI LU NL**

(30) Priorität: **21.06.1989 CH 2311/89**

(43) Veröffentlichungstag der Anmeldung:
**27.12.1990 Patentblatt 1990/52**

(73) Patentinhaber: **CIBA-GEIGY AG**
**CH-4002 Basel (CH)**

(72) Erfinder: **Karrer, Friedrich, Dr.**
**CH-4800 Zofingen (CH)**

(56) Entgegenhaltungen:
**EP-A- 0 004 334**
**EP-A- 0 072 475**
**GB-A- 2 084 574**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.9.9/3.4

EP 0 404 720 B1

## Beschreibung

Die vorliegende Erfindung betrifft 2-[4-(3-Chlorphenoxy)-phenoxy]-äthylcarbaminsäureäthylester, Verfahren zu dessen Herstellung, sowie Mittel welche diesen Wirkstoff enthalten und dessen Verwendung zur Bekämpfung von Reiskulturen schädigenden Zikaden und von Obstschädlingen. Der erfindungsgemäss verwendete Carbaminsäureester ist besonders geeignet zur Bekämpfung von Reis-Zikaden der Familie Delphacidae mit der Gattung Nilaparvata und der Familie Cidadellidae mit der Gattung Nephotettix sowie von Obstschädlingen der Familie Diaspididae mit der Gattung Aonidiella, der Familie Tortricidae mit der Gattung Adoxophyes und der Familie Olethreutidae mit den Gattungen Cydia und Lobesia.

Aus den publizierten Patentanmeldungen EP-A-4 334 und GB-A-2 084 574 sind die Herstellung von substituierten 2-(4-Phenoxyphenoxy)-äthylcarbaminsäure-alkylestern und deren Verwendung zur Bekämpfung pflanzenschädigender Insekten bekannt. Als eine bevorzugte Einzelverbindung wird in der EP-A-4 334 die Verbindung 2-[4-(4-Chlorphenoxy)-phenoxy]-äthylcarbaminsäure-äthylester hervorgehoben.

Demgegenüber wurde nun überraschenderweise gefunden, dass sich der 2-[4-(3-Chlorphenoxy)-phenoxy]-äthylcarbaminsäure-äthylester auch ausgezeichnet zur Bekämpfung von Zikaden, die Reiskulturen befallen, wie zum Beispiel Nilaparvata lugens, Nephotettix cincticeps und Nephotettix virescens sowie zur Bekämpfung verschiedener Obstschädlinge, insbesondere der Wicklerarten wie Adoxophyes reticulana (Fruchtschalenwickler), Cydia pomonella (Apfelwickler) oder Lobesia botrana (Traubenwickler), der Schildläuse in Citruskulturen wie Aonidiella aurantii (Orangenschildlaus) sowie der Weissen Fliegen im Baumwolle und Gemüse wie Bemisia tabaci und Trialeurodes vaporariorum eignet. Der erfindungsgemäss verwendete 2-[4-(3-Chlorphenoxy)-phenoxy]-äthylcarbaminsäure-äthylester ist im wesentlichen als Chemosterilant, beziehungsweise populationshemmende Substanz und als Ovizid wirksam.

Nebst den erwähnten, besonders vorteilhaften Wirkungen besitzt der 2-[4-(3-Chlorphenoxy)-phenoxy]-äthylcarbaminsäureäthylester auch Wirkung gegen die folgenden Schädlinge:
aus der Ordnung Lepidoptera zum Beispiel
Amylois spp., Coleophora spp., Yponomeuta spp., Prays spp., Lyonetia spp., Keiferia lycopersicella, Pectinophora gossypiella, Plutella xylostella, Leucoptera scitella, Lithocollethis spp., Aegeria spp., Synanthedon spp., Adoxophyes spp., Pieris spp., Archips spp., Argyrotaenia spp., Choristoneura spp., Pandemis spp., Sparganothis spp., Cnephasia spp., Acleris spp., Tortrix spp., Cochylis spp., Eupoecilia ambiguella, Hedya nubiferana, Lobesia botrana, Eucosma spp., Cydia spp., Grapholita spp., Pammene spp., Malacosoma spp., Manduca sexta, Chilo spp., Diatraea spp., Crocidolomia binotalis, Ostrinia nubilalis, Cadra cautella, Ephestia spp., Operophtera spp., Thaumetopoea spp., Euproctis spp., Lymantria spp., Agrotis spp., Euxoa spp., Mamestra brassicae, Panolis flammea, Busseola fusca, Sesamia spp., Spodoptera spp., Heliothis spp., Earias spp., Autographa spp., Trichoplusia ni, Cryptophlebia leucotreta, Phthorimaea operculella, Diparopsis castanea, Alabama argillaceae, Anticarsia gemmatalis und Hellula undalis, Cnaphalocrocis spp., Scirpophaga spp., Hyphantria cunea, Carposina nipponensis, Phtorimaea operculella, Cryptophlebia leucotreta, Clysia ambiguella und Pieris rapae;
aus der Ordnung Coleoptera zum Beispiel
Sitotroga spp., Leptinotarsa decemlineata, Diabrotica spp., Agriotes spp., Anthonomus spp., Cosmopolites spp., Dermestes spp., Epilachna spp., Orycaephilus spp., Sitophilus spp., Otierhynchus spp., Tribolium spp., Tenebrio spp., Melolontha spp., Popillia spp., Rhizopertha spp., Trogoderma spp., Curculio spp., Eremnus spp. und Phylctinus spp., Lissorhoptrus spp., Chaetocnema tibialis, Psylliodes spp., Atomaria linearis und Scarabeidae;
aus der Ordnung Orthoptera zum Beispiel
Blatta spp., Periplaneta spp., Leucophaea maderae, Blattella spp., Gryllotalpa spp., Locusta spp. und Schistocerca spp.;
aus der Ordnung Isoptera zum Beispiel
Reticulitermes spp.;
aus der Ordnung Psocoptera zum Beispiel
Liposcelis spp.;
aus der Ordnung Anoplura zum Beispiel
Phylloxera spp., Pemphigus spp., Pediculus spp., Haematopinus spp. und Linognathus spp.;
aus der Ordnung Mallophaga zum Beispiel
Trichodectes spp. und Damalinea spp.;
aus der Ordnung Thysanoptera zum Beispiel
Hercinothrips spp., Thrips tabaci, Taeniothrips spp. und Scirtothrips aurantii, Frank Liniella spp. und Thrips palmi;
aus der Ordnung Heteroptera zum Beispiel
Eurygaster spp., Dysdercus spp., Piesma spp., Cimex spp., Rhodnius spp., Triatoma spp., Nezzara spp., Scotinophara spp., Leptocorisa spp., Euchistus spp., Sahlbergella singularis und Distantiella theobroma;
aus der Ordnung Homoptera zum Beispiel
Aleurodes brassicae, Bemisia tabaci, Trialeurodes vaporariorum, Aphididea, Empoasca spp., Nephotettix spp., Laodelphax spp., Nilaparvata spp., Aonidiella spp., Lecanium corni, Saissetia spp., Aspidiotus spp., Pseudococcus spp., Planococcus spp., Pseudaulacaspis spp., Quadraspidiotus spp., Psylla spp., Chrysomphalus aonidium, Aleurothrixus

floccosus, Trioza erytreae, Eriosoma larigerum, Unaspis citri, Ceroplaster spp. und Partatoria spp., Lepidosaphes spp., Erythroneura spp., Gascardia spp., Coccus hesperidum, Pulvinaria aethiopica, Schizaphis spp., Aphis spp., Sitobion spp., Macrosiphus spp., Rhopalosiphum spp., Myzus spp., Pemphigus spp., Scaphoideus spp. und Chrysophalus dictyospermi;
aus der Ordnung Hymenoptera zum Beispiel
Diprion spp., Hoplocampa spp., Lasius spp., Monomorium pharaonis, Vespa spp., Neodiprion spp. und Solenopsis spp., Atta spp., Acromyrex, Diprionidae, Gilpinia polytoma und Cephus spp.;
aus der Ordnung Diptera zum Beispiel
Aedes spp., Culex spp., Drosophila melanogaster, Musca spp., Fannia spp., Calliphora erythrocephala, Lucilia spp., Chrysomyia spp., Cuterebra spp., Glossina spp., Gastrophilus spp., Hyppobosca spp., Stomoxys spp., Oestrus spp., Hypoderma spp., Tabanus spp., Tannia app., Bibio hortulanus, Oscinella frit, Phorbia spp., Pegomyia hyoscyami, Ceratitis spp., Dacus spp., Tipula spp., Liriomyza spp., Melanagromyza spp., Antherigona soccata, Sciara spp., Rhagoletis pomonella und Orseolina spp.;
aus der Ordnung Siphonaptera zum Beispiel
Xenopsylla cheopis und Ceratophyllus spp.;
aus der Ordnung der Acarina zum Beispiel
Panonychus spp., Tetranychus spp., Tarsonemus spp., Bryobia praetiosa, Acarus siro, Argas spp., Ornithodoros spp., Dermanyssus gallinae, Eriophyes spp., Phyllocoptruta oleivora, Boophilus spp., Rhipicephalus spp., Amblyomma spp., Hyalomma spp., Ixodes spp., Psoroptes spp., Chorioptes spp., Saracoptes spp., Aceria sheldoni, Polyphagotarsonemus latus, Eotetranychus carpini und Brevipalpus spp., Calipitrimerus spp., Aculus schlechtendali, Rhizoglyphus spp. und Olygonychus pratensis und
aus der Ordnung der Thysanura zum Beispiel
Lepisma saccharina.

Ferner kann der 2-[4-(3-Chlorphenoxy)-phenoxy]-äthylcarbaminsäure-äthylester auch zur Bekämpfung von pflanzenschädigenden Nematoden und Schnecken eingesetzt werden.

Der erfindungsgemässe Carbaminsäureester kann analog dem in der EP-A-4 334 beschriebenen Verfahren hergestellt werden.

So erhält man den erfindungsgemässen 2-[4-(3-Chlorphenoxy)-phenoxy]-äthylcarbaminsäureäthylester der Formel I

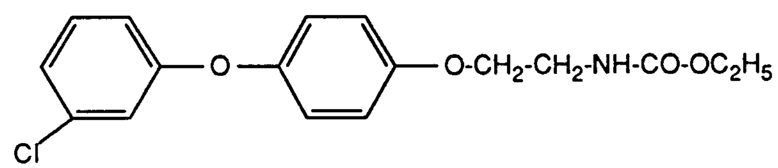

(I)

beispielsweise, indem man

a) 4-(3-Chlorphenoxy)-phenol der Formel II

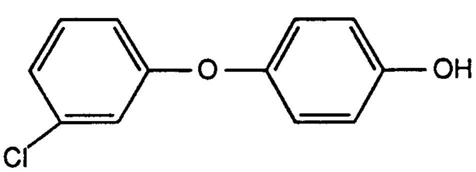

(II)

in Gegenwart einer Base in einem inerten organischen Lösungsmittel mit einem 2-Aethylcarbaminsäure-äthylester der Formel III

$$X\text{-}CH_2\text{-}CH_2\text{-}NH\text{-}CO\text{-}OC_2H_5 \quad \text{(III)}$$

worin X für eine Abgangsgruppe, wie Halogen oder eine Sulfonyloxygruppe, vorzugsweise Chlor, Brom, Methylsulfonyloxy oder p-Tolylsulfonyloxy, steht, umsetzt, oder

b) 2-[4-(3-Chlorphenoxy)-phenoxy]-äthylamin der Formel IV

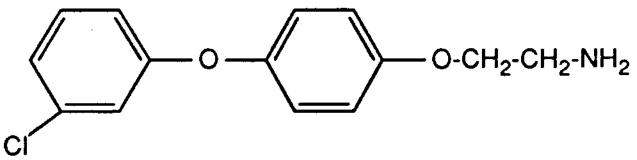

(IV)

in Gegenwart eine Base mit Chlorameisensäureäthylester der Formel V

$$Cl\text{-}CO\text{-}OC_2H_5 \qquad (V)$$

umsetzt.

Als Basen dienen bei der Umsetzung

$$(II + III \rightarrow I)$$

vorzugsweise anorganische Basen wie Alkalicarbonate, Alkalialkoxide, Alkalihydroxide oder Alkalihydride, Alkalibicarbonate, beispielsweise Natriumcarbonat, Kaliumcarbonat, Natriumhydrogencarbonat, Kaliumhydrogencarbonat oder tertiäre Amine. Als Lösungsmittel werden vorzugsweise aprotische polare Lösungsmittel wie Dialkylamide wie Dimethylformamid, ferner Dioxan, Tetrahydrofuran, Dimethoxyäthan, Dimethylsulfoxid oder Sulfolan, oder Mischungen davon eingesetzt. Die Reaktionstemperatur liegt im allgemeinen zwischen -20°C und dem Siedepunkt des Reaktionsgemisches. Vorzugsweise wird eine Reaktionstemperatur über +60°C, insbesondere im Siedebereich des gewählten Lösungsmittels, eingestellt. Als vorteilhaft hat sich der Zusatz einer katalytisch wirkenden Menge eines Alkalijodids, wie Kaliumjodid, erwiesen. In sehr polaren Lösungsmitteln, wie zum Beispiel Dimethylsulfoxid oder Sulfolan, kann in Gegenwart einer oben angegebenen Base zwischen +10°C und +50°C, bevorzugt bei +20°C bis +40°C, gearbeitet werden.

Bei der Umsetzung

$$(IV + V \rightarrow I)$$

kommen als Basen vorzugsweise tertiäre Amine wie Trialkylamine wie z.B. Triäthylamin oder N-Aethyl-diisopropylamin, Pyridin, Dialkylaminopyridine wie Dimethylaminopyridin oder Dialkylaniline wie N,N-Dimethylanilin in Frage. Die Reaktion wird im allgemeinen zwischen -20°C und +100°C durchgeführt. Insbesondere eignet sich der Temperaturbereich zwischen 0°C und + 100°C. Mit Vorteil führt man die Umsetzung in einem organischen Lösungsmittel durch. Geeignetere Lösungsmittel sind zum Beispiel: Aether wie Diäthyläther, Dimethoxyäthan, Tetrahydrofuran oder Dioxan, Kohlenwasserstoffe wie Toluol, Benzol, Xylole, Hexan oder die verschiedenen Benzinfraktionen, oder chlorierte Kohlenwasserstoffe wie Methylenchlorid, Chloroform, Dichloräthan oder Trichloräthan.

Die Ausgangsverbindungen der Formeln II, III, IV und V sind bekannt und teilweise im Handel erhältlich.

Die insektizide Wirkung des 2-[4-(3-Chlorphenoxy)-phenoxy]-äthylcarbaminsäure-äthylesters lässt sich durch Zusatz von anderen Insektiziden und/oder Akariziden wesentlich verbreitern und an gegebene Umstände anpassen. Als Zusätze eignen sich beispielsweise organische Phosphorverbindungen; Nitrophenole und deren Derivate; Formamide; Harnstoffe; pyrethrinartige Verbindungen sowie Carbamate und chlorierte Kohlenwasserstoffe.

Die gute pestizide Wirkung des 2-[4-(3-Chlorphenoxy)-phenoxy]-äthylcarbaminsäure-äthylesters entspricht einer erzielten Abtötungsrate (Mortalität) von mindestens 50-60 % der erwähnten zu bekämpfenden Schadinsekten.

Der erfindungsgemässe 2-[4-(3-Chlorphenoxy)-phenoxy]-äthylcarbaminsäure-äthylester wird in unveränderter Form oder vorzugsweise zusammen mit den in der Formulierungstechnik üblichen Hilfsmitteln eingesetzt und wird daher zum Beispiel zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in verschiedenen polymeren Stoffen in bekannter Weise verarbeitet. Die Anwendungsverfahren wie Versprühen, Vernebeln, Verstäuben, verstreuen oder Giessen werden gleich wie die Art der Mittel den angestrebten Zielen und den gegebenen Verhältnissen entsprechend gewählt.

Die erfindungsgemäss zu verwendenden Formulierungen, das heisst die den insektiziden Wirkstoff und gegebenenfalls einen festen oder flüssigen Zusatzstoff enthaltenden Mittel, Zubereitungen oder Zusammensetzungen werden in bekannter Weise hergestellt, zum Beispiel durch inniges Vermischen und/oder Vermahlen der Wirkstoffe mit Streckmitteln, wie mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyl-äther, Essigester, Propylmyristat oder Propylpalmitat, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; Silikonöle oder Wasser.

Als feste Trägerstoffe, für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien wie Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art der angestrebten Formulierung nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen wie wasserlösliche synthetische oberflächenaktive Verbindungen sein.

Als Seifen eignen sich die Alkali-, Erdalkali- oder gegenbenenfalls substituierten Ammoniumsalze von höheren Fettsäuren ($C_{10}$-$C_{22}$), wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind auch die Fettsäuremethyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel asl Alkali-, Erdalkali-oder gegebenenfalls substituierte Ammoniumsalze vor und weisen einen Alkylrest mit 8 bis 22 C-Atomen auf, wobei Alkyl auch den Alkylteil von Acylresten einschliesst, zum Beispiel das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind beispielsweise die Natrium-, Calcium- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie zum Beispiel Salze des Phosphorsäureesters eines p-Nonylphenol-(4-14)-Aethylenoxid-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5-Aethylenglykoleinheiten.

Als Beispiele nichtionischer Tenside seien Nonylphenolpolyäthoxyäthanole, Ricinusölpolyglycoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Poloxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, zum Beispiel das Stearyltrimethylammoniumchlorid oder das Benzyldi(2-chloräthyl)-äthylammoniumbromid.

Die in der Formulierungstechnik gegräuchlichen Tenside sind u.a. in folgenden Publikationen beschrieben:
"1985 International Mc Cutcheon's Emulsifiers & Detergents", Glen Rock NJ USA, 1985",
H. Stache, "Tensid-Taschenbuch", 2. Aufl., C. Hanser Verlag München, Wien 1981,
M. and J. Ash. "Encyclopedia of Surfactants", Vol. I-III, Chemical Publishing Co., New York, 1980-1981.

Die pestiziden Zubereitungen enthalten in der Regel 0,1 bis 99 %, insbesondere 0,1 bis 95 % des 2-[4-(3-Chlorphenoxy)-phenoxy]-äthylcarbaminsäure-äthylesters, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,01 und 1000 ppm, vorzugsweise zwischen 0,01 und 500 ppm. Die Aufwandmengen pro Hektar betragen im allgemeinen 0,1 bis 1000 g Wirkstoff pro Hektar, vorzugsweise 1 bis 500 g/ha.

Insbesondere setzen sich bevorzugte Formulierungen folgendermassen zusammen: (% = Gewichtsprozent)

| Emulgierbare Konzentrate | |
|---|---|
| Aktiver Wirkstoff: | 1 bis 20 %, bevorzugt 5 bis 10 % |
| oberflächenaktives Mittel: | 5 bis 30 %, vorzugsweise 10 bis 20 % |
| flüssiges Trägermittel: | 50 bis 94 %, vorzugsweise 70 bis 85 % |

| Stäube: | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

| Suspension-Konzentrate | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

| Benetzbare Pulver | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

| Granulate | |
|---|---|
| aktiver Wirkstoff: | 0,5 bis 30 %, vorzugsweise 3 bis 15 % |
| festes Trägermittel: | 99,5 bis 70 %, vorzugsweise 97 bis 85 % |

Die Mittel können auch weitere Zusätze wie Stabilisatoren, Entschäumer, Konservierungsmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Si schränken die Erfindung nicht ein.

Beispiel 1A: Herstellung des 2-[4-(3-Chlorphenoxy)-phenoxy]-äthycarbaminsäure-äthylesters

Zu einer Lösung von 13,2 g 4-(3-Chlorphenoxy)-phenol (Smp. 53-54°C) in 50 ml Dimethylformamid werden 16,6 g feinpulveriges Kaliumcarbonat, 13,6 g 2-Chloräthylcarbaminsäureäthylester und 1 g pulverisiertes Kaliumjodid hinzugefügt. Das Reaktionsgemisch wird unter Rühren während 15 Stunden auf +95°C erwärmt. Hierauf wird das Reaktionsgemisch filtriert und das Filtrat mit Eiswasser aufgenommen und dreimal mit Aether extrahiert. Die organischen Extrakte werden mit Wasser gewaschen, über Natriumsulfat getrocknet und eingedampft. Das erhaltene Rohprodukt wird durch Chromatographie an Kieselgel mit Aether/Hexan-Gemisch (1:3) gereinigt. Man erhält den reinen 2-[4-(3-Chlorphenoxy)-phenoxy]-äthylcarbaminsäureäthylester vom Smp. 45-46°C. Das $^1H$-NMR-Spektrum steht mit der angegebenen Struktur in Einklang.

Beispiel 1B:

Zu einer Lösung von 24,8 g 2-[4-(3-Chlorphenoxy)-phenoxy]-äthylamin und 16,8 g Aethyl-diisopropylamin in 150 ml Toluol lässt man bei +80°C unter Rühren die Lösung von 13 g Chlorameisensäureäthylester in 20 ml Toluol zutropfen und rührt anschliessend weitere 15 Stunden bei dieser Temperatur. Nach dem Abkühlen auf Raumtemperatur wird das Reaktionsgemisch nacheinander mit Wasser, 1N-Salzsäure und schliesslich mit Natriumchloridlösung gewaschen und über Natriumsulfat getrocknet. Das Lösungsmittel wird im Vakuum abdestilliert. Das Rohprodukt wird durch Chromatographie an Kieselgel (Eluierungsmittel: Diäthyläther/n-Hexan, 1:3) weiter gereinigt, wodurch der reine 2-[4-(3-Chlorphenoxy)-phenoxy]-äthylcarbaminsäureäthylester vom Smp. 45-46°C erhalten wird. Die so erhaltene Verbindung ist mit dem nach Beispiel 1A erhaltenen Produkt identisch.

Beispiel 2: Wirkung gegen Nilaparvata lugens

Reispflanzen werden mit einer wässrigen Emulsions-Spritzbrühe, die zwischen 400 ppm und 0,2 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zykaden auf den behandelten zu denjenigen auf den unbehandelten Pflanzen wird die prozentuale Reduktion der Population (% Wirkung) bestimmt.

Der erfindungsgemässe 2-[4-(3-Chlorphenoxy)-phenoxy]-äthylcarbaminsäure-äthylester erzeugt in diesem Test bei 0,2 ppm eine 80-100%ige Wirkung, während der isomere 2-[4-(4-Chlorphenoxy)-phenoxy]-äthylcarbaminsäure-äthylester der EP-A-4 334 diese Wirkung nur bei der Konzentration 400 ppm erreicht.

Beispiel 3: Ovizide Wirkung auf Cydia pomonella

Auf Filterpapier abgelegte Eier von Cydia pomonella werden für eine kurze Zeit in eine acetonisch-wässrige Testlösung, die zwischen 400 ppm und 0,01 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

2-[4-(3-Chlorphenoxy)-phenoxy]-äthylcarbaminsäure-äthylester zeigt in diesem Test noch bei 0,01 ppm 80-100 % Wirkung gegen Cydia pomonella.

Beispiel 4: Ovizide Wirkung auf Adoxophyes reticulana

Auf Filterpapier abgelegte Eier von Adoxophyes reticulana werden für kurze Zeit in eine acetonisch-wässrige Testlösung, die zwischen 400 ppm und 0,05 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

2-[4-(3-Chlorphenoxy)-phenoxy]-äthylcarbaminsäure-äthylester zeigt in diesem Test auch noch bei 0,05 ppm eine 80-100%ige Wirkung geben Adoxophyes reticulana, während der isomere 2-[4-(4-Chlorphenoxy)-phenoxy]-äthylcarbaminsäure-äthylester diese Wirkung nur bei einer Konzentration von ca 50 ppm erreicht.

Beispiel 5: Ovizide Wirkung auf Lobesia botrana

Auf Filterpapier abgelegte Eier von Lobesia botrana werden für kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

2-[4-(3-Chlorphenoxy)-phenoxy]-äthylcarbaminsäure-äthylester zeigt in diesem Test gute Wirkung gegen Lobesia botrana.

Beispiel 6: Wirkung gegen Aonidiella aurantii

Kartoffelknollen werden mit Wanderlarven ("Crawlern") von Aonidiella aurantii (rote Citrus-Schildlaus) besiedelt. Nach etwa 2 Wochen werden die Kartoffeln in eine wässrige Emulsins- resp. Suspensions-Spritzbrühe getaucht, die den zu prüfenden Wirkstoff in einer Konzentration von 400 ppm enthält. Nach dem Abtrocknen der so behandelten Kartoffelknollen werden diese in einem Plastikbehälter inkubiert. Zur Auswertung wird 10-12 Wochen später die Ueberlebensrate der Wanderlarven der ersten Folgegeneration der behandelten Schildlaus-Population mit derjenigen der unbehandelten Kontrollansätze verglichen.

2-[4-(3-Chlorphenoxy)-phenoxy]-äthylcarbaminsäure-äthylester zeigt in diesem Test gute Wirkung gegen Aonidiella aurantii.

Beispiel 7: Wirkung gegen boophilus microplus

Vollgesogene adulte Zeckenweibchen werden auf eine PVC Platte aufgeklebt und mit einem Wattebausch überdeckt. Zur Behandlung werden die Testtiere mit 10 ml einer wässrigen Testlösung, die 125 ppm des zu prüfenden Wirkstoffes enthält übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung.

2-[4-(3-Chlorphenoxy)-phenoxy]-äthylcarbaminsäure-äthylester zeigt in diesem Test gute Wirkung gegen Boophilus microplus.

Beispiel 8: Wirkung gegen Aedes aegypti

50-100 Larven von Aedes aegypti werden in 200 ml einer wässrigen Testlösung, die 400 ppm des zu prüfenden Wirkstoffes und ganz wenig Futter enthält, gegeben. Anschliessend wird das Testgefäss mit einem Deckel verschlossen und inkubiert. Die Auswertung erfolgt 14 Tage nach dem Ansatz auf %-Schlupf der Adulten im Vergleich zu den unbehandelten Kontrollansätzen.

2-[4-(3-Chlorphenoxy)-phenoxy]-äthylcarbaminsäure-äthylester zeigt in diesem Test gute Wirkung gegen Aedes aegypti.

Beispiel 9: Wirkung gegen Nephotettix cincticeps

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

2-[4-(3-Chlorphenoxy)-phenoxy]-äthylcarbaminsäure-äthylester zeigt eine gute Wirkung gegen Nephotettix cincticeps in diesem Test.

Beispiel 10: Wirkung gegen Bemisia tabaci

Buschbohnen-Pflanzen werden in Gazekäfige gestellt und mit Adulten von Bemisia tabaci (Weisse Fliege) besiedelt. Nach erfolgter Eiablage werden alle Adulten entfernt und 10 Tage später die Pflanzen mit den darauf befindenden Nymphen mit einer wässrigen Emulsins-Spritzbrühe der zu prüfenden Wirkstoffe (Konzentration 400 ppm) behandelt. Die Auswertung erfolgt 14 Tage nach der Wirkstoff-Applikation auf %-Schlupf im Vergleich zu den unbehandelten Kontrollansätzen.

2-[4-(3-Chlorphenoxy)-phenoxy]-äthylcarbaminsäure-äthylester zeigt in diesem Test gute Wirkung gegen Bemisia tabaci.

Beispiel 11: Insektizide Formulierungen für den Wirkstoff 2-[4-(3-Chlorphenoxy)-phenoxy]-äthylcarbaminsäure-äthylester (% = Gewichtsprozent)

| 1. Spritzpulver | a) | b) | c) |
|---|---|---|---|
| Wirkstoff der Formel I | 20 % | 50 % | 75 % |
| Na-Ligninsulfonat | 5 % | 5 % | - |
| Na-Laurylsulfonat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kielselsäure | 5 % | 10 % | 10 % |
| Kaolin | 67 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2. Emulsions-Konzentrat | a) | b) |
|---|---|---|
| Wirkstoff der Formel I | 10 % | 10 % |
| Octylphenolpolyäthylenglykoläther (4-5 Mol AeO) | 3 % | - |
| Ca-Dodecylbenzolsulfonat | 3 % | - |
| Ricinusölpolyglykoläther (36 Mol AeO) | 4 % | - |
| Ricinusölthioxilat | - | 25 % |
| Cyclohexanon | 30 % | - |
| Butanol | - | 15 % |
| Xylolgemisch | 50 % | - |
| Essigester | - | 50 % |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff der Formel I | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| 4. Extruder Granulat | |
|---|---|
| Wirkstoff der Formel I | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 5. Umhüllungs-Granulat | |
|---|---|
| Wirkstoff der Formel I | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 6. Suspensions-Konzentrat | |
|---|---|
| Wirkstoff der Formel I | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2% |
| Silikonöl in Form einer 75%igen wässrigen Emulsion | 0,8% |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Patentansprüche

**Patentansprüche für folgende Vertragsstaaten : AT, BE, CH, DE, DK, FR, GB, GR, IT, LI, LU, NL**

1. Die Verbindung 2-[4-(3-Chlorphenoxy)-phenoxy]-äthylcarbaminsäureäthylester der Formel I

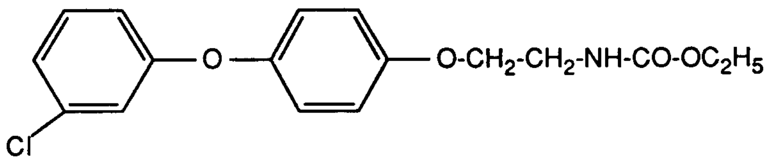

(I).

2. Verfahren zur Herstellung der Verbindung gemäss Anspruch 1, dadurch gekennzeichnet, dass man

   a) 4-(3-Chlorphenoxy)-phenol der Formel II

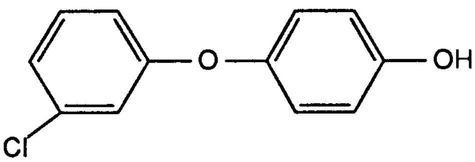

(II)

   in Gegenwart einer Base in einem inerten organischen Lösungsmittel mit 2-Aethylcarbaminsäure-äthylester der Formel III

$$X\text{-}CH_2\text{-}CH_2\text{-}NH\text{-}CO\text{-}OC_2H_5 \qquad (III)$$

   b) 2-[4-(3-Chlorphenoxy)-phenoxy]-äthylamin der Formel IV

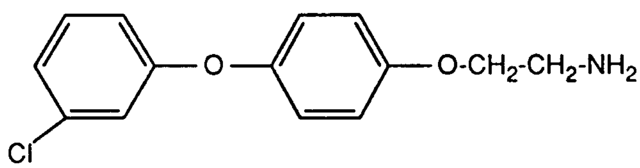

(IV)

   in Gegenwart einer Base mit Chlorameisensäureäthylester der Formel V

$$Cl\text{-}CO\text{-}OC_2H_5 \qquad (V)$$

   umsetzt.

3. Insektizides Mittel, dadurch gekennzeichnet, dass als aktive Komponente 2-[4-(3-Chlorphenoxy)-phenoxy]-äthylcarbaminsäure-äthylester gemäss Anspruch 1 enthält.

4. Verwendung der Verbindung gemäss Anspruch 1 zur Bekämpfung von reisschädigenden Zikaden der Familien Delphacidae und Cicadellidae, vorzugsweise der Gattungen Nilaparvata und Nephotettix und insbesondere von Nilaparvata lugens.

5. Verfahren zur Bekämpfung von Reiskulturen schädigenden Zikaden, dadurch gekennzeichnet, dass man die Schädlinge oder deren verschiedene Entwicklungsstadien oder ihren Aufenthaltsort mit einer pestizid wirksamen Menge

des 2-[4-(3-Chlorphenoxy)-phenoxy]-äthylcarbaminsäure-äthylesters oder mit einem Mittel enthaltend eine pestizid wirksame Menge dieser Verbindung, in Kontakt bringt oder behandelt.

6. Verwendung der Verbindung gemäss Anspruch 1 zur Bekämpfung von Obst-, Citrus-und Weinschädlingen der Familien Diaspididae, Tortricidae oder Olethreutidae, vorzugsweise der Gattungen Aonidiella, Adoxophyes, Cydia oder Lobesia und insbesondere von Cydia pomonella, Adoxophyes reticulana, Lobesia botrana oder Aonidiella aurantii.

7. Verfahren zur Bekämpfung von Obstkulturen schädigenden Wicklern, dadurch gekennzeichnet, dass man die Schädlinge oder deren verschiedene Entwicklungsstadien oder ihren Aufenthaltsort mit einer pestizid wirksamen Menge des 2-[4-(3-Chlorphenoxy)-phenoxy]-äthylcarbaminsäure-äthylesters oder mit einem Mittel enthaltend eine pestizid wirksame Menge dieser Verbindung, in Kontakt bringt oder behandelt.

8. Verfahren zur Bekämpfung von Obstkulturen schädigenden Schildläusen, dadurch gekennzeichnet, dass man die Schädlinge oder deren verschiedene Entwicklungsstadien oder ihren Aufenthaltsort mit einer pestizid wirksamen Menge des 2-[4-(3-Chlorphenoxy)-phenoxy]äthylcarbaminsäure-äthylesters oder mit einem Mittel enthaltend eine pestizid wirksame Menge dieser Verbindung, in Kontakt bringt oder behandelt.

9. Verwendung der Verbindung gemäss Anspruch 1 zur Bekaämpfung von Schädlingen der Familien Noctuidae und Aleyrodidae in Baumwolle un Gemüse, vorzugsweise Gattungen Heliothis, Spodoptera, Bemisia und Trialeurodes und insbesondere von Heliothis virescens, Heliothis zea, Heliothis armigera, Spodoptera littoralis, Spodoptera exigua, Bemisia tabaci oder Trialeurodes vaporariorum.

10. Verfahren zur Bekämpfung von Baumwoll- und Gemüsekulturen schädigenden eulenartigen Nachtfaltern und weissen Fliegen, dadurch gekennzeichnet, dass man die Schädlinge oder deren verschiedene Entwicklungsstadien oder ihren Aufenthaltsort mit einer pestizid wirksamen Menge des 2-[4-(3-Chlorphenoxy)-phenoxy]-äthylcarbaminsäure-äthylesters oder mit einem Mittel enthaltend eine pestizid wirksame Menge dieser Verbindung, in Kontakt bringt oder behandelt.

**Patentansprüche für folgenden Vertragsstaat : ES**

1. Insektizides Mittel, dadurch gekennzeichnet, dass es als aktive Komponente den 2-[4-(3-Chlorphenoxy)-phenoxy]-äthylcarbaminsäureäthylester der Formel I

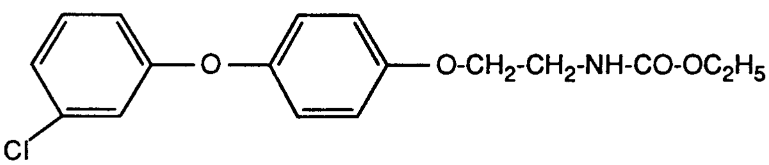

(I)

enthält.

2. Verfahren zur Herstellung des Wirkstofffes der Formel I gemäss Anspruch 1, dadurch gekennzeichnet, dass man

a) 4-(3-Chlorphenoxy)-phenol der Formel II

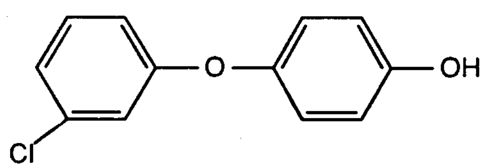

(II)

in Gegenwart einer Base in einem inerten organischen Lösungsmittel mit 2-Aethylcarbaminsäure-äthylester der Formel III

$$X\text{-}CH_2\text{-}CH_2\text{-}NH\text{-}CO\text{-}OC_2H_5 \quad \text{(III)}$$

b) 2-[4-(3-Chlorphenoxy)-phenoxy]-äthylamin der Formel IV

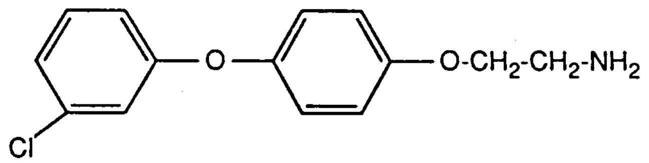

(IV)

in Gegenwart einer Base mit Chlorameisensäureäthylester der Formel V

$$Cl\text{-}CO\text{-}OC_2H_5 \quad (V)$$

umsetzt.

3. Verwendung der Verbindung der Formel I gemäss Anspruch 1 zur Bekämpfung von reisschädigenden Zikaden der Familien Delphacidae und Cicadellidae, vorzugsweise der Gattungen Nilaparvata und Nephotettix und insbesondere von Nilaparvata lugens.

4. Verfahren zur Bekämpfung von Reiskulturen schädigenden Zikaden, dadurch gekennzeichnet, dass man die Schädlinge oder deren verschiedene Entwicklungsstadien oder ihren Aufenthaltsort mit einer pestizid wirksamen Menge des 2-[4-(3-Chlorphenoxy)-phenoxy]-äthylcarbaminsäure-äthylesters oder mit einem Mittel enthaltend eine pestizid wirksame Menge dieser Verbindung, in Kontakt bringt oder behandelt.

5. Verwendung der Verbindung der Formel I gemäss Anspruch 1 zur Bekämpfung von Obst-, Citrus- und Weinschädlingen der Familien Diaspididae, Tortricidae oder Olethreutidae, vorzugsweise der Gattungen Aonidiella, Adoxophyes, Cydia oder Lobesia und insbesondere von Cydia pomonella, Adoxophyes reticulana, Lobesia botrana oder Aonidiella aurantii.

6. Verfahren zur Bekämpfung von Obstkulturen schädigenden Wicklern, dadurch gekennzeichnet, dass man die Schädlinge oder deren verschiedene Entwicklungsstadien oder ihren Aufenthaltsort mit einer pestizid wirksamen Menge des 2-[4-(3-Chlorphenoxy)-phenoxy]-äthylcarbaminsäure-äthylesters oder mit einem Mittel enthaltend eine pestizid wirksame Menge dieser Verbindung, in Kontakt bringt oder behandelt.

7. Verfahren zur Bekämpfung von Obstkulturen schädigenden Schildläusen, dadurch gekennzeichnet, dass man die Schädlinge oder deren verschiedene Entwicklungsstadien oder ihren Aufenthaltsort mit einer pestizid wirksamen Menge des 2-[4-(3-Chlorphenoxy)-phenoxy]äthylcarbaminsäure-äthylesters oder mit einem Mittel enthaltend eine pestizid wirksame Menge dieser Verbindung, in Kontakt bringt oder behandelt.

8. Verwendung der Verbindung der Formel I gemäss Anspruch 1 zur Bekaämpfung von Schädlingen der Familien Noctuidae und Aleyrodidae in Baumwolle und Gemüse, vorzugsweise Gattungen Heliothis, Spodoptera, Bemisia und Trialeurodes und insbesondere von Heliothis virescens, Heliothis zea, Heliothis armigera, Spodoptera littoralis, Spodoptera exigua, Bemisia tabaci oder Trialeurodes vaporariorum.

9. Verfahren zur Bekämpfung von Baumwoll- und Gemüsekulturen schädigenden eulenartigen Nachtfaltern und weissen Fliegen, dadurch gekennzeichnet, dass man die Schädlinge oder deren verschiedene Entwicklungsstadien oder ihren Aufenthaltsort mit einer pestizid wirksamen Menge des 2-[4-(3-Chlorphenoxy)-phenoxy]-äthylcarbaminsäure-äthylesters oder mit einem Mittel enthaltend eine pestizid wirksame Menge dieser Verbindung, in Kontakt bringt oder behandelt.

## Claims

**Claims for the following Contracting States : AT, BE, CH, DE, DK, FR, GB, GR, IT, LI, LU, NL**

1. The compound ethyl 2-[4-(3-chlorophenoxy)phenoxy]-ethylcarbamate of formula I

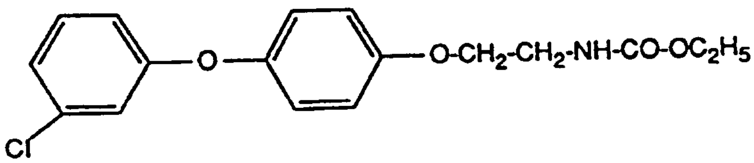

(I).

2. A process for the preparation of the compound according to claim 1, which comprises

   a) reacting 4-(3-chlorophenoxy)phenol of formula II

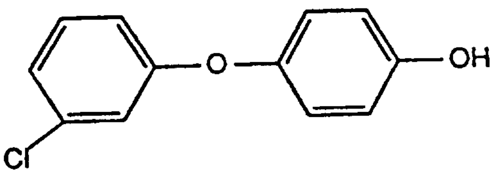

(II)

   in the presence of a base in an inert organic solvent with the ethyl 2-ethylcarbamate of formula III

$$X\text{-}CH_2\text{-}CH_2\text{-}NH\text{-}CO\text{-}OC_2H_5 \qquad \text{(III)}$$

   in which X is a leaving group
   or
   b) reacting 2-[4-(3-chlorophenoxy)phenoxy]ethylamine of formula IV

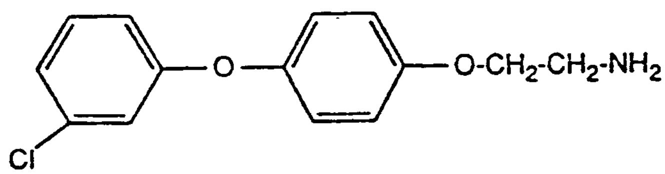

(IV)

   in the presence of a base with ethyl chloroformate of formula V

$$Cl\text{-}CO\text{-}OC_2H_5 \qquad \text{(V)}$$

3. An insecticidal composition which contains ethyl 2-[4-(3-chlorophenoxy)phenoxy]ethylcarbamate according to claim 1 as active component.

4. Use of the compound according to claim 1 for controlling rice-damaging cicadas of the families Delphacidae and Cicadellidae, preferably of the genera Nilaparvata and Nephotettix and, in particular, Nilaparvata lugens.

5. A method of controlling damaging cicadas in rice crops, which comprises contacting or treating said pests or their various development stages or the locus thereof with a pesticidally effective amount of ethyl 2-[4-(3-chlorophenoxy)phenoxy]ethylcarbamate or with a composition comprising a pesticidally effective amount of said compound.

6. Use of the compound according to claim 1 for controlling fruit, citrus fruit and vine pests of the families Diaspididae, Tortricidae or Olethreutidae, preferably of the genera Aonidiella, Adoxophyes, Cydia or Lobesia and, in particular, Cydia pomonella, Adoxophyes reticulana, Lobesia botrana or Aonidiella aurantii.

7. A method of controlling damaging leaf rollers in fruit crops, which comprises contacting or treating said pests or their various development stages or the locus thereof with a pesticidally effective amount of ethyl 2-[4-(3-chlorophenoxy)phenoxy]ethylcarbamate or with a composition comprising a pesticidally effective amount of said compound.

8. A method of controlling damaging scale insects in fruit crops, which comprises contacting or treating said pests or their various development stages or the locus thereof with a pesticidally effective amount of ethyl 2-[4-(3-chlorophenoxy)phenoxy]carbamate or with a composition comprising a pesticidally effective amount of said compound.

9. Use of the compound according to claim 1 for controlling pests of the families Noctuidae and Aleyrodidae in cotton and vegetable crops, preferably the genera Heliothis, Spodoptera, Bemisia and Trialeurodes and, in particular, Heliothis virescens, Heliothis zea, Heliothis armigera, Spodoptera littoralis, Spodoptera exigua, Bemisia tabaci or Trialeurodes vaporariorum.

10. A method of controlling damaging noctuids and white flies in cotton and vegetable crops, which comprises contacting or treating said pests or their various development stages or the locus thereof with a pesticidally effective amount of ethyl 2-[4-(3-chlorophenoxy)-phenoxy]ethylcarbamate or with a composition comprising a pesticidally effective amount of said compound.

**Claims for the following Contracting State : ES**

1. An insecticidal composition which contains ethyl 2-[4-(3-chlorophenoxy)phenoxy]ethylcarbamate of formula I

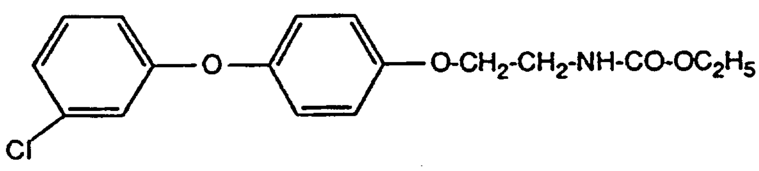

(I)

as active component.

2. A process for the preparation of the active compound of formula I according to claim 1, which comprises

a) reacting 4-(3-chlorophenoxy)phenol of formula II

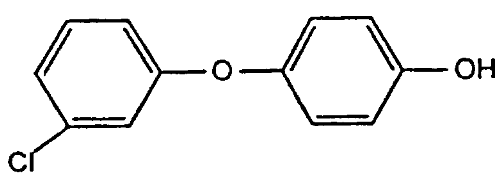

(II)

in the presence of a base in an inert organic solvent with the ethyl 2-ethylcarbamate of formula III

$$X\text{-}CH_2\text{-}CH_2\text{-}NH\text{-}CO\text{-}OC_2H_5 \qquad (III)$$

in which X is a leaving group
or

b) reacting 2-[4-(3-chlorophenoxy)phenoxy]ethylamine of formula IV

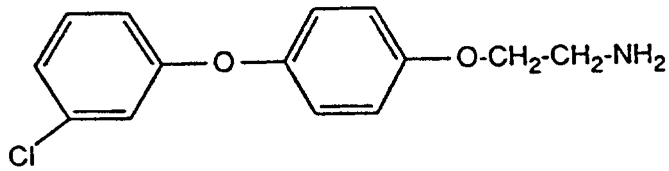

(IV)

in the presence of a base with ethyl chloroformate of formula V

$$Cl\text{-}CO\text{-}OC_2H_5 \qquad (V)$$

3. Use of the compound of formula I according to claim 1 for controlling rice-damaging cicadas of the families Delphacidae and Cicadellidae, preferably of the genera Nilaparvata and Nephotettix and, in particular, Nilaparvata lugens.

4. A method of controlling damaging cicadas in rice crops, which comprises contacting or treating said pests or their various development stages or the locus thereof with a pesticidally effective amount of ethyl 2-[4-(3-chlorophenoxy)phenoxy]ethylcarbamate or with a composition comprising a pesticidally effective amount of said compound.

5. Use of the compound of formula I according to claim 1 for controlling fruit, citrus fruit and vine pests of the families Diaspididae, Tortricidae or Olethreutidae, preferably of the genera Aonidiella, Adoxophyes, Cydia or Lobesia and, in particular, Cydia pomonella, Adoxophyes reticulana, Lobesia botrana or Aonidiella aurantii.

6. A method of controlling damaging leaf rollers in fruit crops, which comprises contacting or treating said pests or their various development stages or the locus thereof with a pesticidally effective amount of ethyl 2-[4-(3-chlorophenoxy)phenoxy]ethylcarbamate or with a composition comprising a pesticidally effective amount of said compound.

7. A method of controlling damaging scale insects in fruit crops, which comprises contacting or treating said pests of their various development stages or the locus thereof with a pesticidally effective amount of ethyl 2-[4-(3-chlorophenoxy)phenoxy]ethylcarbamate or with a composition comprising a pesticidally effective amount of said compound.

8. Use of the compound of formula I according to claim 1 for controlling pests of the families Noctuidae and Aleyrodidae in cotton and vegetable crops, preferably the genera Heliothis, Spodoptera, Bemisia and Trialeurodes and, in particular, Heliothis virescens, Heliothis zea, Heliothis armigera, Spodoptera littoralis, Spodoptera exigua, Bemisia tabaci or Trialeurodes vaporariorum.

9. A method of controlling damaging noctuids and white flies in cotton and vegetable crops, which comprises contacting or treating said pests or their various development stages or the locus thereof with a pesticidally effective amount of ethyl 2-[4-(3-chlorophenoxy)-phenoxy]ethylcarbamate or with a composition comprising a pesticidally effective amount of said compound.

## Revendications

**Revendications pour les Etats contractants suivants : AT, BE, CH, DE, DK, FR, GB, GR, IT, LI, LU, NL**

1. Composé 2-[4-(3-chlorophénoxy)phénoxy]éthylcarbamate d'éthyle de formule I

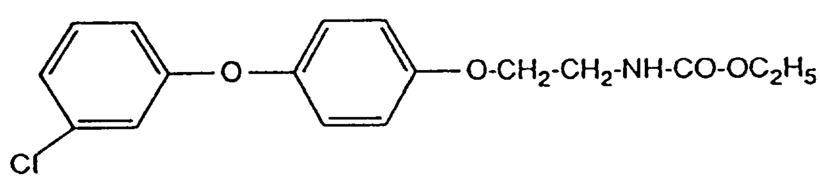

(I).

2. Procédé pour la préparation du composé selon la revendication 1, caractérisé en ce que

a) on fait réagir le 4-(3-chlorophénoxy)phénol de formule II

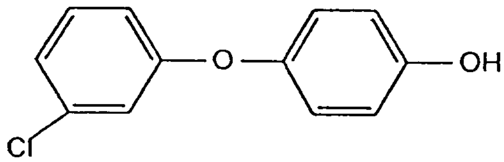

(II)

en présence d'une base, dans un solvant organique inerte, avec le 2-éthylcarbamate d'éthyle de formule III

$$X\text{-}CH_2\text{-}CH_2\text{-}NH\text{-}CO\text{-}OC_2H_5 \qquad \text{(III)}$$

b) on fait réagir la 2-[4-(3-chlorophénoxy)phénoxy]éthylamine de formule IV

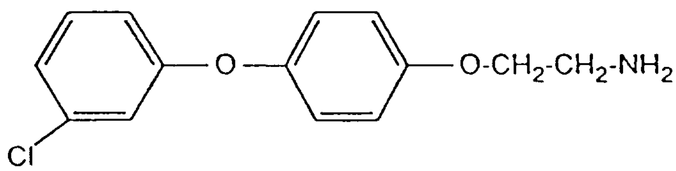

(IV)

en présence d'une base, avec le chloroformiate d'éthyle de formule V

$$Cl\text{-}CO\text{-}OC_2H_5 \qquad \text{(V).}$$

3. Produit insecticide, caractérisé en ce qu'il contient comme composant actif le 2-[4-(3-chlorophénoxy)phénoxy]éthylcarbamate d'éthyle selon la revendication 1.

4. Utilisation du composé selon la revendication 1 pour combattre des cicadidés ravageurs du riz, appartenant aux familles des delphacidés et des cicadellidés, de préférence aux genres *Nilaparvata* et *Nephotettix*, et en particulier *Nilaparvata lugens*.

5. Procédé pour combattre des cicadidés ravageurs de cultures de riz, caractérisé en ce que l'on traite ou met en contact les ravageurs ou leurs divers stades de développement, ou leur habitat, par une quantité à effet pesticide du 2-[4-(3-chlorophénoxy)phénoxy]éthylcarbamate d'éthyle ou par un produit contenant une quantité à effet pesticide de ce composé.

6. Utilisation du composé selon la revendication 1 pour combattre des ravageurs de fruitiers, d'agrumes et de la vigne, appartenant aux familles des diaspididés, tortricidés et oléthreutidés, de préférence aux genres *Aonidiella*, *Adoxophyes*, *Cydia* et *Lobesia*, et en particulier *Cydia pomonella*, *Adoxophyes reticulana*, *Lobesia botrana* et *Aonidiella aurantii*.

7. Procédé pour combattre des tordeuses ravageuses de cultures de fruits, caractérisé en ce que l'on traite ou met en contact les ravageurs ou leurs divers stades de développement, ou leur habitat, par une quantité à effet pesticide du 2-[4-(3-chloropnénoxy)phénoxy]éthylcarbamate d'éthyle ou par un produit contenant une quantité à effet pesticide de ce composé.

8. Procédé pour combattre des cochenilles ravageuses de cultures de fruits, caractérisé en ce que l'on traite ou met en contact les ravageurs ou leurs divers stades de développement, ou leur habitat, par une quantité à effet pesticide du 2-[4-(3-chlorophénoxy)phénoxy]éthylcarbamate d'éthyle ou par un produit contenant une quantité à effet pesticide de ce composé.

9. Utilisation du composé selon la revendication 1, pour combattre des ravageurs appartenant aux familles des noctuidés et des aleyrodidés dans la culture du coton et des légumineuses, appartenant de préférence aux genres

*Heliothis, Spodoptera, Bemisia* et *Trialeurodes* et en particulier *Heliothis virescens, Heliothis zea, Heliothis armigera, Spodoptera littoralls, Spodoptera exigua, Bemisia tabaci* et *Trialeurodes vaporariorum.*

**10.** Procédé pour combattre les noctuelles et mouches blanches ravageuses de cultures de coton et de légumineuses, caractérisé en ce que l'on traite ou met en contact les ravageurs ou leurs divers stades de développement, ou leur habitat, par une quantité à effet pesticide du 2-[4-(3-chlorophénoxy)phénoxy]éthylcarbamate d'éthyle ou par un produit contenant une quantité à effet pesticide de ce composé.

**Revendications pour l'Etat contractant suivant : ES**

**1.** Produit insecticide, caractérisé en ce qu'il contient, en tant que composant actif, le 2-[4-(3-chlorophénoxy)phénoxy]éthylcarbamate d'éthyle de formule I

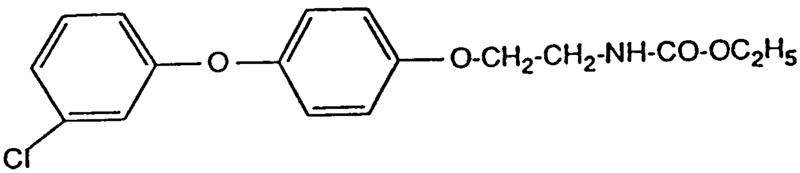

(I)

**2.** Procédé pour la préparation de la substance active de formule I selon la revendication 1, caractérisé en ce que

a) on fait réagir le 4-(3-chlorophénoxy)phénol de formule II

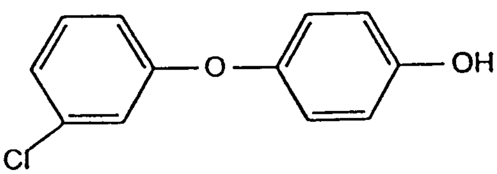

(II)

en présence d'une base, dans un solvant organique inerte, avec le 2-éthylcarbamate d'éthyle de formule III

$$X-CH_2-CH_2-NH-CO-OC_2H_5 \qquad (III)$$

b) on fait réagir la 2-[4-(3-chlorophénoxy)phénoxy]éthylamine de formule IV

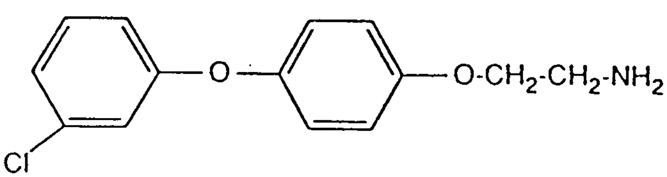

(IV)

en présence d'une base, avec le chloroformiate d'éthyle de formule V

$$Cl-CO-OC_2H_5 \qquad (V).$$

**3.** Utilisation du composé de formule I selon la revendication 1 pour combattre des cicadidés ravageurs du riz, appartenant aux familles des delphacidés et des cicadellidés, de préférence aux genres *Nilaparvata* et *Nephotettix*, et en particulier *Nilaparvata lugens.*

**4.** Procédé pour combattre des cicadidés ravageurs de cultures de riz, caractérisé en ce que l'on traite ou met en contact les ravageurs ou leurs divers stades de développement, ou leur habitat, par une quantité à effet pesticide

du 2-[4-(3-chlorophénoxy)phénoxy]éthylcarbamate d'éthyle ou par un produit contenant une quantité à effet pesticide de ce composé.

5. Utilisation du composé de formule I selon la revendication 1 pour combattre des ravageurs de fruitiers, d'agrumes et de la vigne, appartenant aux familles des diaspididés, tortricidés et olethreutidés, de préférence aux genres *Aonidiella*, *Adoxophyes*, *Cydia* et *Lobesia*, et en particulier *Cydia pomonella*, *Adoxophyes reticulana*, *Lobesia botrana* et *Aonidiella aurantii*.

6. Procédé pour combattre des tordeuses ravageuses de cultures de fruits, caractérisé en ce que l'on traite ou met en contact les ravageurs ou leurs divers stades de développement, ou leur habitat, par une quantité à effet pesticide du 2-[4-(3-chlorophénoxy)phénoxy]éthylcarbamate d'éthyle ou par un produit contenant une quantité à effet pesticide de ce composé.

7. Procédé pour combattre des cochenilles ravageuses de cultures de fruits, caractérisé en ce que l'on traite ou met en contact les ravageurs ou leurs divers stades de développement, ou leur habitat, par une quantité à effet pesticide du 2-[4-(3-chlorophénoxy)phénoxy]éthylcarbamate d'éthyle ou par un produit contenant une quantité à effet pesticide de ce composé.

8. Utilisation du composé de formule I selon la revendication 1, pour combattre des ravageurs appartenant aux familles des noctuidés et des aleyrodidés dans la culture du coton et des légumineuses, appartenant de préférence aux genres *Heliothis*, *Spodoptera*, *Bemisia* et *Trialeurodes* et en particulier *Heliothis virescens*, *Heliothis zea*, *Heliothis armigera*, *Spodoptera littoralis*, *Spodoptera exigua*, *Bemisia tabaci* et *Trialeurodes vaporariorum*.

9. Procédé pour combattre les noctuelles et mouches blanches ravageuses de cultures de coton et de légumineuses, caractérisé en ce que l'on traite ou met en contact les ravageurs ou leurs divers stades de développement, ou leur habitat, par une quantité à effet pesticide du 2-[4-(3-chlorophénoxy)phénoxy]éthylcarbamate d'éthyle ou par un produit contenant une quantité à effet pesticide de ce composé.